# EUROPEAN PATENT APPLICATION

(11) **EP 0 850 615 A1**
(43) Date of publication of application: **01.07.1998**
(21) Application number: 96120568.9
(22) Date of filing: 20.12.1996
(51) Int. Cl.: A61F 13/15, A61L 15/60

(54) **A dry laid structure comprising particulate material**

(71) Applicant: THE PROCTER & GAMBLE COMPANY, Cincinnati, Ohio 45202 (US)
(72) Inventor: Gagliardi, Ivano, 65123 Pescara (IT); Carlucci, Giovanni, 66100 Chieti (IT)
(74) Representative: Hirsch, Uwe Thomas

(57) **Abstract**

The present invention relates to a dry laid fibrous structure for absorbing aqueous fluids and/or for providing odour control. The fibrous structure comprises a dry laid fibrous web and a particulate material distributed in the web and are bonded by a thermoplastic polymeric material in finely divided form distributed therein; the fibrous structures are particularly suitable for use in disposable absorbent articles.

## Description

### FIELD OF THE INVENTION

The present invention relates to dry laid fibrous structures preferably intended for absorbing aqueous fluids and/or for providing odour control. The structures comprise a dry laid fibrous web and a particulate material distributed in the web and are bonded by a thermoplastic polymeric material distributed therein; they are particularly suitable for use in disposable absorbent articles.

### BACKGROUND OF THE INVENTION

Fibrous structures, particularly fibrous structures for absorbing liquids are manufactured for many uses, for example they are incorporated into absorbent articles such as disposable diapers, incontinent pads and catamenial napkins as fluid absorption or fluid transmission and/or diffusion elements, for example as absorbent cores that are intended to absorb and retain body fluids. Fibrous structures, and more specifically fibrous structures used in absorbent articles as fluid absorption or fluid transmission and/or diffusion elements, usually comprise a multiplicity of components so as to improve their specific performances; for example, absorbent structures that comprise fibres and a particulate material, such as an absorbent gelling material in particle form, are known in the art. Further components can be also included to provide the structure with added benefits.

Dry laying and, more specifically, air laying processes are widely used to produce webs from dry fibres, which can in turn be used e.g. as structures for absorbing fluids. Particularly, dry laying refers to the formation of carded webs, i.e., webs in which the fibres are oriented (carded) in a given direction, whereas the air laying process refers to the formation of webs with a completely random fibre orientation; the properties of such air laid webs are therefore somewhat isotropic. The fibrous webs produced by dry laying processes are soft, flexible and porous, and are particularly suitable for use as liquid absorbent structures in absorbent articles, such as disposable diapers, sanitary napkins, incontinent pads, and wipes.

Methods for incorporating further components e.g. in particle form in a dry laid fibrous web are also known in the art; particularly, in US Patent No. 4,765,780 a process and apparatus is described for forming air laid fibrous webs having a multiplicity of components, such as a two layer absorbent core with one layer having an absorbent gelling material in particle form homogeneously blended therein, the other layer being substantially free of absorbent gelling material particles. Similar techniques can be used for incorporating in a dry laid fibrous web different types of components, e.g. an odour control means in particle form in order to provide the absorbent structure constituted by the dry laid fibrous web with the further benefit of odour control.

The dry laid manufacturing process generally comprises a web formation and layering step and a web bonding and stabilizing step; in dry laying processes in fact the fibres, that can be of any type, e.g. cellulosic, synthetic, or any combination thereof, are formed or condensed into a web. Further components that are not in fibre form can also be incorporated in the fibrous web, e.g. a particulate material. The resulting web lacks integrity after formation, and must therefore be stabilized. Different techniques for bonding and stabilizing a dry formed web are known in the art, i.e. mechanical, thermal and chemical bonding processes. Bonding a web structure by means of a chemical is one of the most common methods of bonding in the nonwoven industry, and consists in the application of a chemical binder to the web and in the curing of the binder. The most widely used chemical is latex, since it is cheap, versatile, easy to apply, and very effective as a binder. Several methods are known to apply the latex binder to the fibrous web, while spray bonding and print bonding are particularly preferred for fibrous webs intended to be used in absorbent articles.

European application EP-A-592 001 describes a nonwoven fabric bonded by means of e.g. a latex binder, comprising "particles" or "globules" of a thermoplastic composition arranged in a discontinuous pattern onto at least one of its surfaces, and with an odour control material in powder form affixed to the thermoplastic "particles" or "globules" on the surface of the nonwoven fabric. All the odour control material is affixed to the thermoplastic "particles" in this case and is present only onto the surface of the nonwoven structure.

In European application EP-A-463 716 a dry laid absorbent structure is described that comprises a fibrous web with an absorbent gelling material therein and is stabilized by application of a latex coating to at least a surface of the web.

Thermal bonding process is also widely used in order to bond a dry laid web; such a web comprises fusible fibres such as bicomponent fibres as the sole component fibre, or as a mixture with non fusible fibres, e.g. natural fibres. They are successively caused to melt by heat treatment so as to bond the web structure.

Thin, layered absorbent structures described in International applications WO 94/01069 and WO 95/17868 comprise typically two outer independently formed fibrous layers, typically air laid cellulose tissue layers, and an intemediate layer comprising particles of absorbent gelling material and particles of thermoplastic polymeric material, the two fibrous layers being bonded by the melting of the thermoplastic particles. The fibrous layers are themselves already formed and bonded when the layered structure is manufactured, and therefore constitute two distinct and separate layers joined to form the layered structure, with the particulate material distributed only between the fibrous layers.

Both chemical and thermal bonding processes, and their possible combination as well, suffer of certain disadvantages if applied to dry laid structures comprising a particulate material therein, especially when the particulate material incorporated in the dry laid structure is concentrated preferably intermediate the thickness of the web, therefore forming a region of the web in which the fibres constitute a lower percentage. In such cases a latex composition can hardly penetrate the thickness of the web in order to reach the particles, unless it is used in such an amount that it will impair the performances of the entire structure and, possibly, will cause negative interactions with the particulate material. On the other hand the use of meltable fibres, e.g. bicomponent fibres, with subsequent thermal treatment does not solve the problem since fibres may not be distributed among the particles in such an amount to perform an effective bonding action; moreover, owing to the different nature of fibres as compared to particulate material, a homogeneous distribution of fibres among particulate material is rather difficult to achieve when the particulate material constitute the higher percentage of the mixture. Heat meltable fibres such as bicomponent fibres are also rather expensive.

It is therefore an object of the present invention to provide a dry laid fibrous structure, preferably an air laid fibrous structure which comprises a dry laid fibrous web and a particulate material and has good integrity combined with softness. The dry laid fibrous structure of the present invention can be used as absorbent structure for absorbing fluids, preferably with the further capability of controlling odours which are related to the absorbed fluids.

It has surprisingly been found that a thermoplastic polymeric material in finely divided form, preferably in particle or powder form, can be effectively distributed among the particulate materials and at least partially among the fibres of the dry laid web. The subsequent thermal treatment melts the thermoplastic polymeric material and therefore creates a framework of discrete bonding points within the web, i.e. among the fibres and the particulate material where the thermoplastic polymeric material in finely divided form has been distributed, without substantially modifying the effectiveness of the particulate material itself owing to the very small dimension of the thermoplastic particles. The use of thermoplastic polymeric material in finely divided form can be also combined with a traditional latex bonding, e.g. the thermoplastic powder performs the bonding preferably of the inner portion of the dry laid web, while the application of a reduced amount of latex stabilizes the outer surfaces of the dry laid web. Alternatively, the dry laid web can be entirely bonded by means of thermoplastic polymeric material in finely divided form dispersed uniformly within the whole web and then caused to melt by thermal treatment.

### SUMMARY OF THE INVENTION

The present invention relates to a dry laid fibrous structure; the dry laid fibrous structure comprises a dry laid fibrous web having a first surface and a second surface aligned approximately opposite to the first surface and a particulate material distributed in the web. The dry laid fibrous structure further comprises a thermoplastic polymeric material in finely divided form distributed therein in order to bond the particulate material to the fibres of the dry laid fibrous web.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a schematic, fragmentary side elevational view of an apparatus for making a fibrous structure according to the present invention;
FIG. 2 is an enlarged, cross-sectional view of a fibrous structure according to the present invention;
FIG. 3 is a schematic, fragmentary side elevational view of an apparatus for making an alternate embodiment of a fibrous structure according to the present invention;
FIG. 4 is an enlarged, cross-sectional view of an alternate embodiment of a fibrous structure according to the present invention.

### DETAILED DESCRIPTION OF THE INVENTION

The present invention relates to a dry laid fibrous structure preferably intended for absorbing aqueous fluids and/or for providing odour control. The structure is constituted by a dry laid fibrous web comprising a particulate material and is bonded by a thermoplastic polymeric material distributed therein in finely divided form. In a preferred embodiment, the structures of the present invention are incorporated into absorbent articles, preferably as absorbent structures that are intended to absorb and retain the various body fluids. Absorbent articles, and more specifically disposable absorbent articles, refer to articles such as sanitary napkins, disposable diapers, incontinent pads, that are worn by a user adjacent to the body and are intended to absorb and contain the various body fluids that are discharged from the body (e.g., vaginal discharges, menses, sweat, and/or urine) and which is intended to be discarded after a single use.

The term "use", as used herein, refers to the period of time that starts when the absorbent article is actually put in contact with the anatomy of the user.

In a preferred embodiment of the present invention the dry laid fibrous structures according to the present invention can constitute integrally the absorbent core of a disposable absorbent article, or they can be comprised therein as part of the absorbent core, or in any case they can constitute an element of a disposable absorbent article, e.g. intended for absorption of body fluids, or for odour control, or for both.

Disposable absorbent articles, such as for example sanitary napkins, pantiliners, incontinent pads, or diapers, typically comprise a fluid pervious topsheet, a fluid impervious backsheet, that can optionally be water vapour and/or gas pervious, and an absorbent core comprised therebetween.

The dry laid fibrous structures of the present invention comprise a particulate material distributed in the web that is typically capable of performing absorption of aqueous fluids and/or control of the odours, e.g. those odours associated with the absorbed fluids. Preferably the particulate material comprises an absorbent gelling material and an odour control means, both in particle form.

The fibrous structures of the present invention can be made using conventional equipment designed for dry laying processes, and although the invention is described hereinbelow with particular reference to air laid structures, it should be understood that other dry laying processes, e.g. carding, are also applicable.

The invention will be described as an air laid fibrous structure which is capable of providing absorption of aqueous fluids, particularly body fluids, and control of the odours associated with the absorbed fluids, being intended to be incorporated as absorbent structure in a disposable absorbent article, e.g. a sanitary napkin.

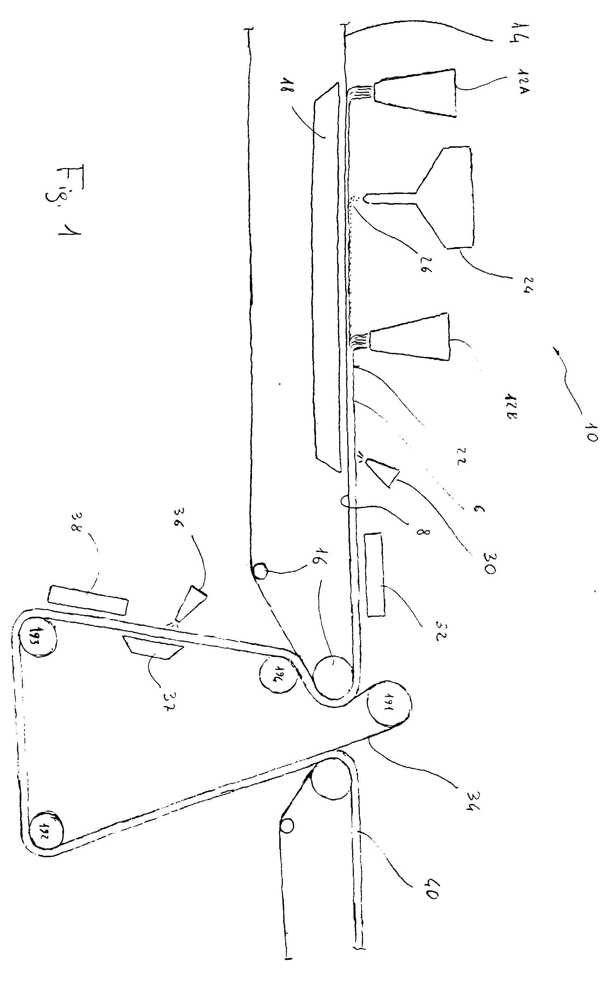

FIG. 1 is a simplified schematic illustration of a preferred embodiment for the manufacture of the fibrous structure of the present invention. In accordance with this embodiment, the air forming system, indicated generally by the numeral 10, includes a distributor unit 12 disposed transversely above a continuous forming screen 14 mounted on rollers 16 and driven by a suitable motor (not shown), and vacuum means or suction box 18 is positioned beneath the screen. In a conventional air forming system, upstream of the distributor unit is a defibrator or feeder (not shown), such as a hammermill or Rando-Feeder, where bales, laps or the like are defiberized, and further the fibres may be cleaned and/or blended if necessary or desired depending largely on the type of fibres used, the blend of fibres used, and the end product sought. For example, wood pulp fibres can be blended with synthetic fibres and applied as a blend by a single distributor, or different fibres can be each conveyed by a different distributor to the screen to form separate plies or layers.

The porous forming screen 14 is essentially coextensive with the distributors, and the suction box 18 beneath the screen draws the air stream downwardly and conveys the fibres to the surface of the screen thereby forming plies or a loose web 22. At this stage in the process, the web exhibits little integrity, and the vacuum means retains the loose, fibrous web on the screen. The web 22 has a first surface 6 that faces the distributor and a second surface 8, opposite to surface 6, that faces the forming screen 14.

It should be understood that the system may be modified to control the composition and thickness of the end product. For example, the distributor unit can comprise a plurality of individual distributors, and although FIG. 1 shows schematically two distributors at 12A and 12B, this number of distributors and particular arrangement can be altered or varied depending on such factors as machine speed, capacity, type of fibres, and end product desired.

Web 22 formed on screen 14 has incorporated therein a particulate material. In a preferred embodiment the particulate material comprises a mixture of an absorbent gelling material and of an odour control means, both in particle or powder form. In a preferred embodiment as shown in FIG. 1, a dosing unit or feed hopper 24, containing the particulate material is positioned in the middle of the distributor unit, i.e. between distributors 12A and 12B. In the embodiment of FIG. 1 the dosing unit 24 receives the different particulate materials from supply containers 25 and 26, which respectively contain the absorbent gelling material and the odour control means in particle form. The dosing unit 24 preferably provides the particulate material in a homogeneous mixture.

A thermoplastic polymeric material in finely divided form, preferably in powder form, is also added to the fibrous web 22; in a preferred embodiment the thermoplastic polymeric material in powder form is supplied to the dosing unit 24 from the container 27, and is homogeneously mixed with the particulate material coming from the containers 25 and 26.

In this manner, the particulate material comprising the thermoplastic polymeric material in powder form are deposited between plies of fibres laid by each distributor. That is, the particulate material and the thermoplastic polymeric material are discharged from hopper 24 onto the moving layer of fibres laid down by distributor 12A, and the ply of fibres laid down by distributor 12B is laid over the particulate material. It should be understood, however, that the plies are relatively porous, and therefore the particulate material and the thermoplastic polymeric material tend to distribute somewhat within adjacent plies. Therefore the resulting fibrous web 22 comprises the particulate material and the thermoplastic polymeric material concentrated intermediate the thickness of the web, forming a region of the web 22 in which the fibres constitute a lower percentage as compared to the particulate material and the thermoplastic polymeric material. Where desired, the particulate material may be blended with the fibres in one or more distributors, such as in distributor 12A or 12B, thereby forming a web with particulate material intermixed with one or more fibrous plies of the web. It is however preferred that the particulate material with the thermoplastic polymeric material is distributed within the thickness of the web 22, intermediate the first and second surfaces 6, 8.

The web 22 condensed on forming wire 14 has very little integrity and requires stabilization. In the embodiment shown in FIG. 1 the web is subjected to a first stabilization step by means of thermal treatment at a bonding station 28. The particulate material and the fibres of the web 22 that are comprised among the particulate material and, at least partially, in the two plies, are bonded together by the application of heat and, optionally, of moderate pressure to melt the thermoplastic polymeric material in powder form mixed with the particulate material constituted by the absorbent gelling material and by the odour control means.

The bond among the particulate material and the fibres is generated by the melting of the individual particles of thermoplastic polymeric material in powder form; as it melts, the thermoplastic polymeric material forms "bridges" connecting directly the particulate material and the fibres.

The overall surface area of the bond points represents a small fraction of the surface area of the particulate material and of the fibres that are involved in the bonding, the characteristics of which thus remain almost unchanged. Since the thermoplastic polymeric material in the preferred powder form can be homogeneously distributed among the particulate material and the fibres comprised therein it can therefore provide the web 22 with an effective bonding within the portion interested by the distribution of the thermoplastic polymeric material itself: in the embodiment of the present invention achieved by the apparatus of FIG. 1 this corresponds to the region of the web 22 mainly constituted by the particulate material with a lesser percentage of fibres, intermediate the first and second surfaces 6 and 8.

The absorbent gelling material, which is preferably distributed in the form of particles, may be made of inorganic or organic substances such as cross-linked polymers, all known from the prior art.

The odour control means can be any suitable odour control agent known in the art, or any mixture thereof, for example it can be constituted by particles of zeolite and silica.

The average dimensions of the particulate material, given as a weighted average of the smallest dimensions of the individual particles, can be between 50 microns and 1500 microns, preferably between 100 microns and 800 microns.

The thermoplastic polymeric material in finely divided form, e.g. in form of powder has the purpose of bonding the particulate material and, at least partially, the fibres of the dry laid absorbent structure together by melting and forming discrete, spaced-apart bond points among the particles and the fibres. The thermoplastic polymeric material can also be used in other finely divided forms, e.g. in form of fibrils.

As explained above, the bridges which form these bond points can involve the particulate material and the fibres of the web 22.

The quantity of thermoplastic polymeric material in finely divided form incorporated in the web 22 can be between 5 g/m² and 180 g/m².

The thermoplastic polymeric material in finely divided form can preferably be melted at such a temperature that it does not interfere with the characteristics of the other components of the absorbent structure, i.e. the fibres and the particulate material, which comprises the absorbent gelling material and the odour control means in the preferred embodiment. Therefore, the thermoplastic polymeric material must have fluidity characteristics such as to enable the necessary bonds to be formed rapidly.

These preferred characteristics can be achieved by a thermoplastic polymeric material in finely divided form having a melt flow index (M.F.I.), evaluated by the ASTM method D 1238-85 under conditions 190/2.16, of at least 25 g/10 min, preferably at least 40 g/10 min, and even more preferably at least 60 g/10 min.

If the fibres of the dry formed fibrous structure are short cellulose fibres, it is preferable to use a thermoplastic polymeric material composed of powder of high-density polyethylene with maximum dimensions of the particles of about 400 microns, characterized by a melt flow index of about 50 g/10 min, in a quantity between 12 g/m² and 90 g/m²

According to the embodiment of the present invention illustrated in FIG. 1 the web 22 can be further bonded on one, or preferably both surfaces 6 and 8 by means of the application of a latex composition. The web 22 can be first passed between compression rollers (not shown), which may be heated, to densify the web, but this step is optional. This densification step can enhance the penetration of the latex into the web, and the degree or percent of densification can vary depending on such factors as the amount of odour control particles, basis weight of the web, the desired degree of penetration of the latex into the web, and the end product sought.

After the bonding station 28 and the (optional) compression rollers the web is transported to a suitable dispensing means 30, such as a spray nozzle, doctor blade, roller applicator, or the like, where a latex binder is applied to the first surface 6 of the web 22. A vacuum applied by a suction box 19 positioned beneath the dispensing means and the screen 14 helps to draw the latex into the web. The dispensing means or applicator is essentially coextensive with the width of the web, and preferably a substantially uniform coating is applied to the web surface. However, the latex may be applied as a non uniform, random or pattern coating, and because the latex is water-based, it will diffuse throughout the web and function as a binder when cured.

The latex when cured imparts integrity to the web, and therefore some penetration of the latex is required. The extent or degree of penetration of the latex into the web is controlled by controlling the amount of latex applied and by controlling the vacuum applied to the web in that the vacuum helps to draw the latex into the web. Such extent or degree of penetration can be limited to the surface of the web 22, since the portion of the web 22 comprising the particulate material which is intermediate the surfaces 6 and 8 is already bonded by the thermoplastic polymeric material, and therefore any possible negative interference between the latex composition and the particulate material is avoided. The amount of the latex composition is also kept to such an extent that it does not impair the absorbency and softness characteristics of the fibrous web 22.

The latex is usually applied as an aqueous emulsion, and can be a thermosetting plastic. In order to activate the latex, the latex emulsion contains a suitable curing agent or cross-linking agent, and after the web is coated, the latex is cured to effect cross-linking. Most typically, curing is accomplished by passing the coated web through a hot air oven or through an air drier 32, and the temperature typically ranges from about 100 °C to 260 °C, but this depends upon the specific type of latex resin used, upon the curing agent or cross-linking agent, upon the amount of latex, the thickness of the web, the degree of vacuum, and the machine speed.

It is desirable to coat the second surface 8 of the web 22 with latex as well, and this is readily accomplished by the dispensing means 36 as the web 22 is conveyed on the second screen 34 operating about pulleys 191, 192, 193, and 194. The second dispensing means 36 includes a suction box 37. This second latex coating is likewise cured by passing the web through a second oven 38 within about the same temperature range.

The resulting absorbent structure 40 exiting from the last oven now exhibits sufficient integrity and can be cut, rolled, packaged, etc.

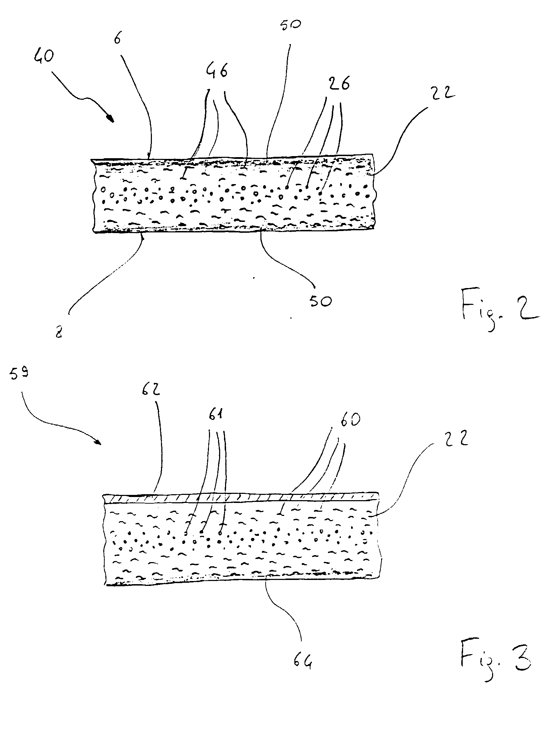

The absorbent structure 40 made in accordance with the foregoing process is illustrated in FIG. 2. The absorbent structure 40 comprises randomly distributed fibres 46, such as wood pulp fibres, and particulate material 42 is distributed in the absorbent structure. The thermoplastic polymeric material 48 in powder form is distributed mainly among the particulate material 42 and performs the bonding of the particulate material and of at least part of the fibres of the absorbent structure 40, i.e. those fibres that enter in contact with the thermoplastic polymeric material. It will be observed that the particulate material is more concentrated in the middle zone of the absorbent structure, but some particles migrate to other sections of the absorbent structure. Both first and second surfaces 6 and 8 of the absorbent web 22 bear a latex coating 50, indicated in the drawing by a shading, which has penetrated or impregnated the absorbent structure to some degree and has partially coated some of the fibres. As explained above, the penetration is controlled so as not to impair the characteristics of the particulate material.

The fibrous structure of the present invention is soft yet strong and absorbent, exhibiting a relatively high tensile strength. It can be desirable for preferred absorbent fibrous structures of this type to have relatively low bulk, because a more dense absorbent structure, when compared to similar structures containing no latex and of about equal absorptive capacity but of higher bulk, can be thinner yet highly absorbent and consequently less bulky. A reduction in bulk, which means a reduction in volume the absorbent fibrous structure is occupying, without sacrificing significantly other desired properties is important from the standpoint of manufacturing, storage and packaging. Hence, for products of the present invention the basis weight can range from about 50 g/m² to 600 g/m², and preferably from about 75 g/m² to 400 g/m², and more preferably from about 250 g/m² to 350 g/m². There can be manufacturing constraints in producing an absorbent structure having a basis weight lower than about 50 g/m² in that such an absorbent structure may lack desired strength. When the basis weight exceeds the upper limit, the product may be too stiff and therefore not useful for most applications.

Any of a variety of fibres, including a blend or admixture, can be used in the fibrous structure of this invention. The fibres may be cellulosic, modified cellulosic, or synthetic, and include such fibres as wood pulp, rayon, cotton, cellulose acetate, polyester, polyethylene, polypropylene, nylon, and the like. A fibrous structure comprising cellulosic fibres such as wood pulp fibres is particularly useful as an absorbent structure in such products as sanitary napkins, disposable diapers or wipes because the cellulose is liquid absorbent and therefore enhances the overall absorbency of the structure. Products of this type, i.e., fibrous structures that are also absorbent, also advantageously use a blend of cellulosic and synthetic fibres, typically comprising about 65% to 95% by weight of cellulosic fibres, and more preferably up to about 20% by weight of the synthetic fibres. The synthetic fibres, which can be provided in any length including staple length, can improve the strength of the structure. They can also be treated to make them hydrophilic, in order not to decrease the absorbent capacity of the preferred absorbent fibrous structure.

Preferred fibrous structures described so far comprise hydrophilic fibres that are substantially absorbent towards aqueous fluids, said structures being useful as absorbent structures in disposable absorbent articles. Dry laid fibrous structures according to the present invention can also comprise hydrophobic fibres only, e.g. synthetic fibres. Such structures can comprise for example as particulate material an odour control means only, being therefore capable of odour control without absorbing and retaining liquid. Such type of structures can be comprised in disposable absorbent articles as a liquid receiving and transmitting layer, e.g. as an acquisition layer comprised between the topsheet and the absorbent core, which is capable of acquiring body fluid quickly and of transmitting it to the absorbent core, performing at the same time an odour control action towards the fluid. A structure according to the present invention comprising hydrophobic synthetic fibres only can also be useful in different applications, e.g. as a filter medium.

Thus, the type of fibres and the particular blend can be varied depending upon the end product. In addition to the foregoing uses, the absorbent structures of this invention can be suitably used for incontinent pads, diaper core, diaper insert, and for surgical and wound bandages, providing absorbent capacity and/or odour control.

The absorbent gelling material, which preferably constitutes at least part of the particulate material comprised in the dry laid absorbent structure of the present invention, may comprise anyone of the well known materials (sometimes referred to as "super-sorbers") that are becoming broadly used in absorbent articles. AGM's are materials which have fluid-absorbing properties. Such materials form hydrogels on contact with water (e.g., with urine, blood, and the like). One highly preferred type of hydrogel-forming, absorbent gelling material is based on polyacids, especially polyacrylic acid. Hydrogel-forming polymeric materials of this type are those which, upon contact with fluids (i.e., liquids) such as water or body fluids, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

The odour control means preferably included in the fibrous structure of the present invention can comprise a wide variety of odour control agents, in order to control unpleasant odours, e.g. those odours associated with the absorbed fluids when the fibrous structure is a fluid absorbent structure.

In their preferred use as absorbent cores in disposable absorbent articles the absorbent structure of the present invention are intended to absorb body fluids.

Various body fluids contain malodorous chemical compounds including acyclic and cyclic amines, aldehydes, fatty acids, and sulfur containing compounds such as sulfides. For example vaginal discharges and used sanitary napkins can contain many malodorous chemical compounds, for example, trimethylamine, pyridine, furaldehyde, isovaleric acid, and methyl mercaptan. The particular malodorous compounds which will be absorbed by various absorbent articles will vary depending upon the person who is wearing the absorbent article and the type of body fluid absorbed, i.e., urine, menstrual fluid, vaginal discharges, perspiration, milk, etc. For feminine pads, such as sanitary napkins or pantiliners, the length of time the article is worn, the quantity of fluid which is absorbed and the exposure of the pad to different body fluids will determine which odours can be emitted by the absorbent article.

Any suitable odour-control agent known in the art can be incorporated in the dry laid fibrous structures of the present invention, to provide the structure with the benefit of odour control, e.g. towards those odours associated with absorbed body fluids when the dry laid fibrous structure is an absorbent structure preferably incorporated into disposable absorbent articles.

Suitable odour-control agents that can be employed in the practice of the present invention can be for example water-soluble antibacterial compounds. Such compounds include, for example, halogenated phenylene compounds (U.S. Patent 3,093,546), periodic acids (U.S. Patent 3,804,094), various copper compounds, especially copper acetate (U.S. Patent 4,385,632), various quaternary ammonium salts, which are well known for their antibacterial properties, e.g. cetyl pyridinium chloride, and the like. Alternatively, antibacterial compounds can be used conjointly with various particulate materials which, in use and in the presence of moisture, release the antibacterial agent. Zeolite materials, such as zeolites which are bactericidal by virtue of having absorbed therein and thereon various bactericidal cations such as copper, silver and zinc, can be advantageously used in the practice of this invention (U.S. Patent 4,525,410). In a preferred mode, the odour control agent is a water-insoluble particulate odour absorbing material such as chlorophyll particles, activated carbon granules, charcoal, ion exchange resin (Japanese 87019865), activated alumina, and absorbent zeolite materials, including the well known "molecular sieve" zeolites of the type A and X and the zeolite materials marketed under the trade name ABSCENTS by the Union Carbide Corporation and UOP, and which are typically available as a white powder in the 3-5 micron particle size range.

In a known mode, the odour-control agent can be a water-insoluble particulate odour-absorbing material such as chlorophyll particles, activated carbon granules, charcoal, ion exchange resin (Japanese 87019865), activated alumina, and absorbent zeolite materials, including the well known "molecular sieve" zeolites of the type A and X and the zeolite materials marketed under the trade name ABSCENTS by the Union Carbide Corporation and UOP, and which are typically available as a white powder in the 3-5 micron particle size range.

The odour-control agents used in the present invention can also comprise other compounds such as cyclodextrin, chelating agents, parabens, chitin, pH buffered materials, silica gel, clays, diatomaceous earth, polystyrene derivatives, starches, and the like. For example, cheating agents as those described in European Applications EP 96109178.2 and EP 96109179.0, both applications filed on 7 June 1996, are particularly preferred. Some partially neutralized hydrogel forming absorbent gelling materials, such as polyacrylate gelling material and acrylate grafted starch gelling material can be also used, preferably in combination with other odour-control agents.

Further odour control agents can comprise acidic compounds such as ascorbic acid, stearic acid, boric acid, maleic acid polymers, malonic acid, maleic acid, polyacrylic acid and monopotassium phosphate, or basic compounds such as inorganic salts of carbonates, bicarbonate, phosphate, biphosphate, sulfate, bisulfate, borate, and mixtures thereof, as those described in US 5037412, or as the combination of boric acid and sodium tetraborate described in International application WO 94/25077.

It is to be understood that the odour-control means that can be preferably employed in the practice of the present invention is not, simply, the odour-control agent, per se, added to the fibrous structure. Rather, the odour-control means comprises any combination of odour-control agents and, optionally, of other materials such as binders. Agglomerates of different odour control agents, e.g. with a binder, can therefore also be used, such as for example an agglomerate of zeolite and silica in particle form, as that described in European application EP 96109175.8, filed on 7 June 1996. The odour-control agent, on the other hand, is the specific odour-control compound.

Preferably, odour control agents are used in the present invention in particulate form. Particularly preferred are odour control means comprising zeolite, silica, preferably in form of silica gel, absorbent gelling material, and combinations thereof, such as the odour control means described in International application WO 95/26207, and in European applications EP 96109177.4, EP 96109173.3, EP 96109174.1, EP 96109176.6.

For example, a preferred odour control means can be a combination of particles of silica gel, zeolite and absorbent gelling material. The weight ratio of absorbent gelling material to silica to zeolite is preferably in the range of from 1:5:1 to 1:1:5, preferably from 1:3:1 to 1:1:3, most preferably from 1:1:1 to 1:1.5:1.5.

It has been found that the odour control material can be incorporated into the fibrous structures according to the present invention in an amount that ranges from 20 g/m² to 400 g/m², preferably from 100 g/m² to 300 g/m², more preferably from 150 g/m² to 250 g/m², with reference to the total surface area of the fibrous structure. Preferably the fibrous structures of the present invention can comprise from 20% to 80% by weight of the odour control means. The weight of the odour control means that can be actually used in various fibrous structures intended for different uses can be readily determined by the skilled person bearing in mind the size and the type of the fibrous structure, and its intended use.

The latex is applied as an aqueous emulsion or dispersion, which typically contains about 45% to 65% solids, and these materials are readily available from several manufacturers. Because the latex emulsions are water miscible, they may be further diluted, if desired, before being applied to the web. Also, these latex compositions are thermosetting, and in order to effect cross-linking, they contain a small amount of a suitable cross-linking agent which are well known chemical agents for this purpose, such as N-methylolacrylamide. Any type of latex known in the art which is suitable for fibrous structures of the present invention can be used, provided that it preferably does not generate detectable odours, especially after curing, since this could be unpleasant for a user and, moreover, could exhaust at least partially a preferred odour control capacity of the fibrous structure well before its intended use. Latices available are classified by chemical family, and those particularly useful include vinyl acetate and acrylic ester copolymers, ethylene vinyl acetate copolymers, styrene butadiene carboxylate copolymers, and polyacrylonitriles, and sold, for example, under the trade names of Airbond, Airflex and Vinac of Air Products, Inc., Hycar and Geon of Goodrich Chemical Co., and Fulatex of H. B. Fuller Company. The amount of latex used in the absorbent structure cannot be so high as to substantially impair or obscure the effective odour control capacity of the odour control means, and also the absorbent properties of the hydrophilic fibres, or as to impart a stiffness to the structure as to render it impractical. It has been found that the latex may range from about 5% to 30% by weight of the structure, and preferably from about 10% to 20% by weight.

Fibrous structures made according to the present invention exhibit a good integrity due to the binding performed by the thermoplastic polymeric material comprised within the structure in finely divided form and, optionally, to the latex coating, and yet have preferably either a remarkable absorbent capacity or an odour control capability, or more preferably both of them, since the particulate material comprised in the fibrous web is affected by the binding means, i.e. by the thermoplastic polymeric material in finely divided form and, possibly, by the latex coating, only to a minimum extent. In case a latex binder is used the depth of penetration of the latex into the fibrous web can controlled by the vacuum applied by means of the suction boxes positioned in correspondence with the dispensing means, and by the choice of the amount to be applied to the web.

Fig. 3 shows a simplified, schematic illustration of an apparatus for making an alternate preferred embodiment of a dry laid fibrous structure 59 according to the present invention, for example an absorbent structure, which structure is in turn illustrated in FIG. 4.

The apparatus of FIG. 3 is similar to the apparatus illustrated in FIG. 1, and same numerals indicate corresponding parts, but in this case the latex applying and curing section is missing, since the whole bonding of the structure is performed by the thermoplastic polymeric material in finely divided form incorporated in the web.

As can be seen from FIG. 3 the thermoplastic polymeric material, preferably in powder form, contained in the container 27 is supplied both to the feeder 24 and to the distributors 12A and 12B. The thermoplastic polymeric material is therefore homogeneously mixed with the particulate material and with the fibres that form the fibrous plies as well.

During the subsequent bonding stage performed at the binding station 28 the whole dry laid structure 59 is thermally stabilized in its entire thickness by the melting of the powder of the thermoplastic polymeric material that bonds both the particulate material comprised intermediate the first and second surfaces 6 and 8, and the fibres of the web 22.

Referring to FIG. 4, the dry laid absorbent structure 59 comprises fibres 60 and particulate material 61 constituted by a mixture of an absorbent gelling material and of an odour control means interspersed in the web, but more concentrated in the middle of the thickness of the web 22. The thermoplastic polymeric material 63 in powder form is homogeneously distributed within the whole fibrous web forming the dry laid absorbent structure 59.

In further alternate embodiments of the present invention either the absorbent gelling material or the odour control means can be included in the absorbent structure in a form different from the particulate form, e.g. fibrous absorbent gelling materials can be used, or odour control means in form of a solution sprayed onto the absorbent structure can be incorporated, provided that a particulate material is comprised in the absorbent structure. For example, a suitable solution could be sprayed onto the first fibrous ply laid down by the distributor 12A in the apparatus illustrated in FIG. 1, or onto the first surface 6 of the web 22 of FIG. 3, instead of, or in combination with, the odour control means in particle form distributed by the hopper 24 within the web 22.

In a further alternate embodiment of the present invention, not illustrated, a latex coating could be applied also to the first ply of the fibrous web, and subsequently cured, before the application of the particulate material, onto the surface where the particulate material is to be distributed.

One of the plies of the fibrous web, for example the second ply of the web of FIG. 3, can also, in another alternate embodiment, be substituted by a nonwoven layer, or by a polymeric film. In this latter case the polymeric film should be applied to the web after the bonding station 28, in order to avoid that the temperature in the bonding station can possibly melt the polymeric film. A structure of this type could constitute an absorbent element of a disposable absorbent article having an impervious layer incorporated therein.

## Claims

1. A dry laid fibrous structure, preferably a dry laid absorbent fibrous structure, said structure comprising a dry laid fibrous web having a first surface and a second surface aligned approximately opposite said first surface, and comprising a particulate material distributed in said web, said dry laid fibrous structure being characterized in that it further comprises a thermoplastic polymeric material in finely divided form distributed therein in order to bond said particulate material to the fibres of said dry laid fibrous web.

2. A dry laid fibrous structure according to claim 1, characterized in that said particulate material comprises an absorbent gelling material.

3. A dry laid fibrous structure according to any preceding claim, characterized in that said particulate material comprises an odour control means.

4. A dry laid fibrous structure according to any preceding claim, characterized in that said particulate material is distributed within the thickness of said dry laid fibrous web, intermediate said first and said second surface.

5. A dry laid fibrous structure according to any preceding claim, characterized in that said thermoplastic polymeric material is in particle or powder form.

6. A dry laid fibrous structure according to any preceding claim, characterized in that said thermoplastic polymeric material is mixed with said particulate material.

7. A dry laid fibrous structure according to any preceding claim, characterized in that said dry laid fibrous structure further comprises latex coating on at least one of said first or said second surfaces of said fibrous web.

8. A dry laid firous structure according to any preceding claim, characterized in that said thermoplastic polymeric material further provides bonding of said dry laid fibrous web.

9. A dry laid fibrous structure according to any preceding claim, characterized in that said dry laid fibrous structure is preferably air laid.

10. A disposable absorbent article comprising a dry laid fibrous structure according to any preceding claim.
